# EUROPEAN PATENT SPECIFICATION

(11) **EP 3 120 749 B1**
(45) Date of publication and mention of the grant of the patent: **21.05.2025**
(21) Application number: 15764629.0
(22) Date of filing: 23.02.2015
(51) Int. Cl.: A61B 1/00, G02B 6/38, A61M 25/01, A61B 5/00, A61L 29/06

(54) **OPTICAL IMAGE DIAGNOSTIC DEVICE AND CATHETER USED IN SAME**
OPTISCHE BILDDIAGNOSEVORRICHTUNG UND DARIN VERWENDETER KATHETER
DISPOSITIF DE DIAGNOSTIC D'IMAGE OPTIQUE ET CATHÉTER ASSOCIÉ

(30) Priority: 19.03.2014 JP 2014057097
(43) Date of publication of application: 25.01.2017
(73) Proprietor: Terumo Kabushiki Kaisha, Tokyo 151-0072 (JP)
(72) Inventor: SAKAGUCHI, Yuuki, Fujinomiya-shi Shizuoka 418-0015 (JP); WATANABE, Kenji, Fujinomiya-shi Shizuoka 418-0015 (JP)
(74) Representative: Casalonga
(86) International application number: PCT/JP2015/000845
(87) International publication number: WO 2015/141134

(56) References cited:
- EP-A1- 1 459 780
- WO-A1-2009/154103
- JP-A- 2009 123 508
- JP-A- 2009 123 508
- JP-A- 2009 184 351
- JP-A- 2009 184 351
- US-A1- 2003 077 043
- US-A1- 2012 002 928
- US-A1- 2012 190 974
- US-A1- 2013 012 810

## Description

### Technical Field

The present invention relates to an imaging apparatus for diagnosis using optical coherence comprising a catheter.

### Background Art

In particular, the present invention relates to an optical imaging apparatus according to the preamble of claim 1, such as it is, e.g., known from US2012/0002928.

There are known, as apparatuses for acquiring an image of a vascular lumen, an optical coherence tomography (OCT: Optical Coherence Tomography) apparatus, etc. Furthermore, as an improved version of the OCT apparatus, the development of a swept-source OCT (SS-OCT) apparatus using wavelength sweep is underway (Patent Literature 1). These are generically referred to as "imaging apparatuses for diagnoses", hereinafter.

The imaging apparatus for diagnosis employs a catheter accommodating an imaging core that transmits and receives light in a tip end portion thereof so as to be rotatable and axially movable. The imaging core is connected to a drive shaft having generally the same length as an overall length of the catheter. Furthermore, the drive shaft accommodates therein an optical fiber. Moreover, a connector to connect the catheter to an MDU (motor drive unit) part included in the imaging apparatus for diagnosis is provided on a rear end of the catheter. The MDU part is a drive unit, which functions as a power source for the rotation and axial movement of the drive shaft, and has a connector to connect the MDU part to the catheter.

The connection between the MDU part and the catheter will now be considered in more detail.

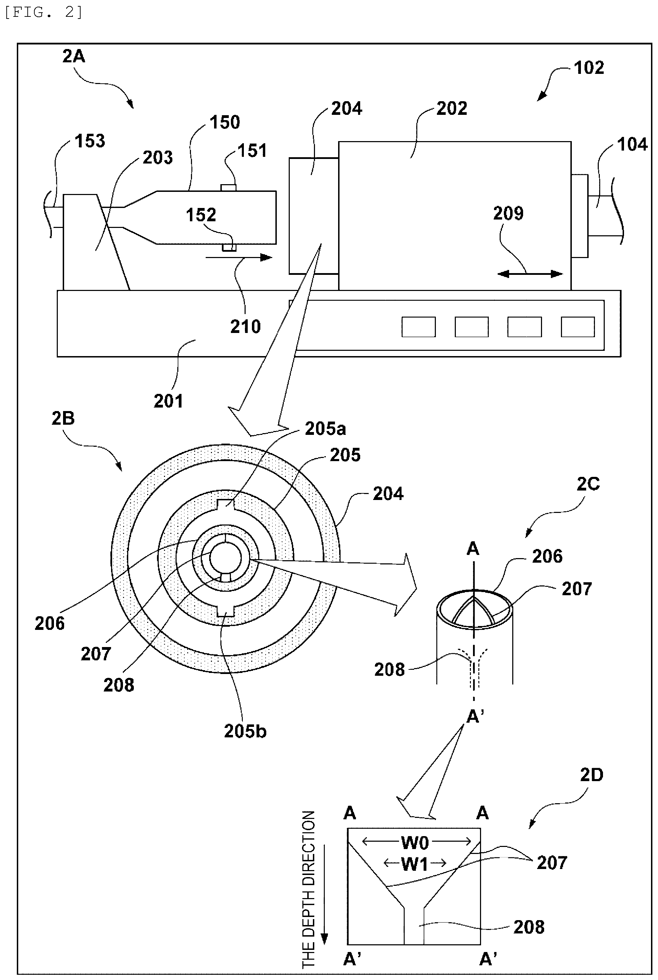

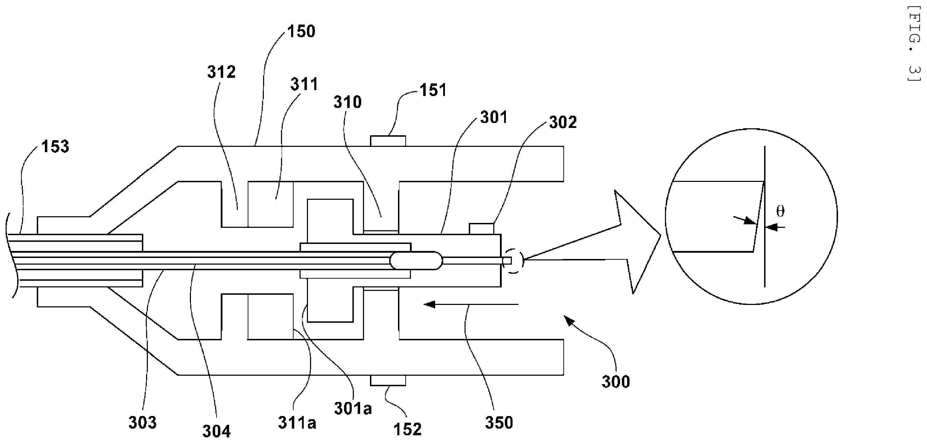

Reference symbol 2A of FIG. 2 illustrates a manner in which a catheter connector section (hereinafter, catheter connector) 150 is moved in the direction of an arrow 210 and connected to a connector section (hereinafter, MDU connector) 204 of the MDU part 102. Reference symbol 2B of FIG. 2 is a front view of the MDU connector 204. Reference symbol 2C of FIG. 2 is a partially transparent perspective view of a knob engagement portion 206 which is located at the center of the MDU connector 204, into which a knob of the catheter is fitted, and which supports the knob thereof. Reference symbol 2D of FIG. 2 is a planar development view illustrating an inner surface of the knob engagement section 206 shown in FIG. 2C when the knob engagement portion 206 is taken along line A-A'. Furthermore, FIG. 3 illustrates a cross-sectional structure of an ordinary catheter connector 150.

The MDU part 102 is roughly composed by a base section 201 and a slider section 202 which is slidable with respect to the base section 201 along an arrow 209 shown in FIG. 2. The base section 201 is provided with various operation switches including a grip section 203 for gripping a catheter sheath 153. Furthermore, the base section 201 has therein a circuit board and a motor for allowing the slider section 202 to slide along the arrow 209. The slider section 202 is provided with the MDU connector 204 accommodating the catheter connector 150 and connected thereto, and has therein a motor for rotating the drive shaft within the catheter and a structure for optically connecting the optical fiber of the drive shaft to an optical fiber within a cable 104. The MDU connector 204 functions as a cover protecting a connection state when the catheter connector 150 is connected to the MDU connector 204.

As indicated by reference symbol 2B of FIG. 2, the MDU connector 204 has therein a connector engagement portions 205 which has guide grooves 205a and 205b for guiding projection portions 151 and 152 which the catheter connector 105 has. When the projection portions 151 and 152 of the catheter connector 150 are fitted into the MDU connector 204 along these guide grooves 205a and 205b, the catheter and the MDU part 102 are mechanically integrated with each other. It is noted that the connector engagement portion 205 does not rotate but is fixed.

Furthermore, when a knob 301 (see FIG. 3) holding the optical fiber located at a central position of the catheter connector 150 is fitted into the knob engagement portion 206, the knob engagement portion 206 is optically connected to the optical fiber within the catheter and rotates integrally with the knob 301. Guide walls 207 for guiding a projection portion 302 provided on a tip end of the knob 301 are provided inside of the knob engagement portion 206. As indicated by reference symbol 2C of FIG. 2, the guide walls 207 correspond to ridgelines of a mountain within the knob engagement portion 206. It is reference symbol 2D of FIG. 2 that a planar development view which illustrates the inner surface of the knob engagement portion 206 taken along line A-A' in the perpendicular direction passing through a peak of the "mountain" defined by the guide walls 207. As clear from 2D of FIG. 2, a space sandwiched between the guide wall 207 forms a guide groove 208. A width WO of the guide groove 208 near an opening side of the knob engagement portion 206 is substantially equal to an inner circumference of the knob engagement portion 206, the width WO changes to a width W1 along the depth direction, and the width in a state where the knob 301 has been fitted into the knob engagement portion 206 to some extent becomes equal to a width of the projection portion 302, thereby guiding the knob 301 linearly. Moreover, an inclination angle of the guide walls 207 is not limited to a specific angle but is preferably 15 degrees to 60 degrees, more preferably 20 degrees to 45 degrees. As this inclination angle is greater, phase shift correction becomes possible. On the other hand, as the inclination angle is greater, a stroke (length in the depth direction) necessary to attach the catheter connector 150 becomes larger, so that an appropriate angle is determined as the inclination angle in the light of design. It is noted that the inclination angle of 30 degrees is adopted.

As shown in FIG. 3, the catheter connector 150 supports a catheter sheath 153 and has an opening portion 300 for connecting the catheter connector 150 to the MDU part 102. The catheter connector 150 has therein the knob 301 which is rotatable integrally with a drive shaft 303 accommodated in the catheter sheath 153. This knob 301 is constituted by PBT (Poly Butylene Terephthalate), and forms a structure for guiding an optical fiber 304 accommodated in the drive shaft and exposing a tip end portion of the optical fiber 304. It is noted that a support section 310 prevents the knob 301 from projecting outside and applies a force to the knob 301 in the direction of cancelling the fitting of the optical connector to the knob 301 when the catheter connector 150 is pulled out from the MDU part 102.

As shown, a surface of an end portion of the optical fiber 304 is not a surface orthogonal to the axial direction thereof but is a slope inclined at an angle θ with respect to the orthogonal surface. On the other hand, an end surface (not shown) of an optical fiber located deep in the knob engagement portion 206 within the MDU part 102 has the same inclination angle, the optical fiber is exposed, and the end surfaces of the two optical fibers are surface-connected to each other. The reason for setting the inclination angle θ to each fiber end surface is to mitigate the influence of reflected light on a connection surface therebetween.

As such, when the catheter connector 150 is attached to the MDU part 102, the respective fiber end surfaces are surface-connected to each other. To be more exact, it is necessary to connect the catheter connector 150 to the MDU part 102 without causing phase shift in the rotational direction. For this reason, the knob 301 has the projection portion (key) 302 for defining an angle of the knob in the rotational direction thereof within the catheter connector 150. On the other hand, the knob engagement portion 206 of the MDU part 102 side is provided with the guide groove 208 defined by the aforementioned guide walls 207 so that the knob 301 and the knob engagement portion 206 are fixed to each other at a determined angle. Namely, when a user holds the catheter connector 150 and inserts the projection portions 151 and 152 of the catheter connector into the connector engagement portion 205 of the MDU connector 204 along the guide grooves 205a and 205b, the projection portion 302 of the knob 301 collides against the guide walls 207 of the knob engagement portion 206, and when the user subsequently and continuously presses the catheter connector 150 into the connector engagement portion 205, the knob engagement portion 206 rotates and the projection portion 302 of the knob 301 is finally guided along the guide groove 208 of the knob engagement portion 206, thereby surface-connecting the end surfaces of the optical fibers of the catheter connector 150 and the MDU part 102 to each other.

As described above, the user performs the operation for holding the catheter connector 150 in user's hand and pressing the catheter connector 150 into the connector section 204 of the MUD part 102. At this time, positions of the projection portions 151 and 152 of the catheter connector 150 are defined in the MDU part 102 side. On the other hand, the knob 301 receives a force for connecting the catheter connector 150 to the MDU part 102 via an elastic body 311 in the catheter insertion direction and is connected to the knob engagement portion 206. At this time, because of many members associated with the connection, it is considered that the knob 301 collides against the knob engagement portion 206 and the connection cannot be completed, depending on a variation in tolerance. In that case, providing the elastic body 311 that tends to relatively deform in an end portion of the knob 301 makes it possible to mitigate dimensional inconsistency during the connection. It is noted that it is necessary for a reaction force when the elastic body 311 deforms to exceed a load necessary for completing the connection between the knob 301 and the knob engagement portion 206; thus, the elastic body 311 has a spring constant equal to or higher than a certain value. A support section 312 is provided to fixedly support the elastic body 311.

Providing the abovementioned structure enables the optical fibers to be appropriately connected to each other when the catheter is connected to the MDU part 102. It is noted that silicone rubber is normally used for this elastic body 311. Furthermore, when the connection between the catheter connector 150 and the MDU connector 204 is completed both mechanically and optically, the knob 301 is integrated with the knob engagement portion 206. The projection portions 151 and 152 are similarly fixedly supported in the MDU part 102. As a result, a sufficient space is created between a rear end surface 301a of the knob 301 and a surface 311a of the elastic body 311.

### Citation List

### Patent Literature

Patent Literature 1: JP-A 2007-267867

### Summary of the Invention

### Technical Problem

As described above, when the user performs the operation for connecting the catheter to the MDU part 102, the projection portion 302 of the knob 301 abuts the guide walls of the knob engagement portion 206 of the MDU part 102. When the user subsequently and continuously performs the operation for pressing the catheter connector 150 into the MDU connector 204, the rear end surface 301a of the knob 301 abuts the surface 311a of the elastic body 311 and a frictional force is generated therebetween. Namely, the knob 301 is suppressed from rotating.

A configuration related to rotation drive within the MDU part 102 is connected to the knob engagement portion 206 of the MDU part 102; therefore, a force for suppressing the rotation (hereinafter, a rotation suppression force for the knob engagement portion 206) acts on the knob engagement portion 206 even in a non-driven state although the knob engagement portion 206 is rotatable. Therefore, when the user continues the operation for inserting the catheter connector 150 in a state where the rear end surface 301a of the knob 301 abuts the surface 311a of the elastic body 311, a considerable load is applied to a contact position between the projection portion 302 of the knob 301 and the guide walls 207 of the knob engagement portion 206, and the projection portion 302 of the knob 301 is broken according to circumstances. On the other hand, because the knob engagement portion 206 of the MDU part 102 is supposed to be used a plurality of times and a resin having sufficiently high hardness is used therefor, the probability of breaking the knob engagement portion 206 is low but yet the probability of flawing a collision position is not zero. Once there occurs a flaw in the position, the knob engagement portion 206 moves jerkily in the position and loses a smooth guiding function, with the result that the connector of the MDU part 102 might be broken according to circumstances.

The present invention has been made in the light of the abovementioned problems and an object of the present invention is to provide a technique for alleviating not only a burden on a catheter but also a burden on an MDU part and for yet enabling the long-term use of the MDU part.

### Means for Solving the Problems

To solve the abovementioned problems, an optical imaging apparatus for diagnosis of the present invention has the configuration according to claim 1. Preferred embodiments are presented in the dependent claims.

### Advantage of the Invention

According to the present invention, it is possible to alleviate not only a burden on the catheter but also a burden on the MDU part and to yet ensure the long-term use of the MDU part with a simple structure.

Other features and advantages of the present invention will become clearer from the disclosure given below with reference to the accompanying drawings. It is noted that same or like configurations are denoted by the same reference symbols in the accompanying drawings.

### Brief Description of the Drawings

The accompanying drawings are encompassed in the specification, constitute part of the specification, illustrate embodiments of the present invention, and are used for describing the principle of the present invention together with the disclosure.
[FIG. 1] FIG. 1 is an external view of an imaging apparatus for diagnosis according to an embodiment.
[FIG. 2] FIG. 2 is a view illustrating a cross-sectional structure of a conventional catheter connector.
[FIG. 3] FIG. 3 is a view illustrating a manner in which an MDU part is connected to a catheter and a structure of a tip end of a connector of the MDU part.
[FIG. 4A] FIG. 4A is a cross-sectional structural view of a catheter connector according to a first embodiment.
[FIG. 4B] FIG. 4B is a perspective view of a ring member in the catheter connector according to the first embodiment.
[FIG. 4C] FIG. 4C is a perspective view of the ring member in the catheter connector according to the first embodiment.
[FIG. 5A] FIG. 5A is a view illustrating a problem to be solved by an illustrative example not falling under the scope of the claims and illustrating a cross-sectional structure of a catheter connector according illustrative example not falling under the scope of the claims.
[FIG. 5B] FIG. 5B is a view illustrating the problem to be solved by an illustrative example not falling under the scope of the claims and illustrating the cross-sectional structure of the catheter connector according to the illustrative example not falling under the scope of the claims.

### Mode for Carrying Out the Invention

Embodiments according to the present invention will be described in detail hereinafter with reference to the accompanying drawings.

### [First Embodiment]

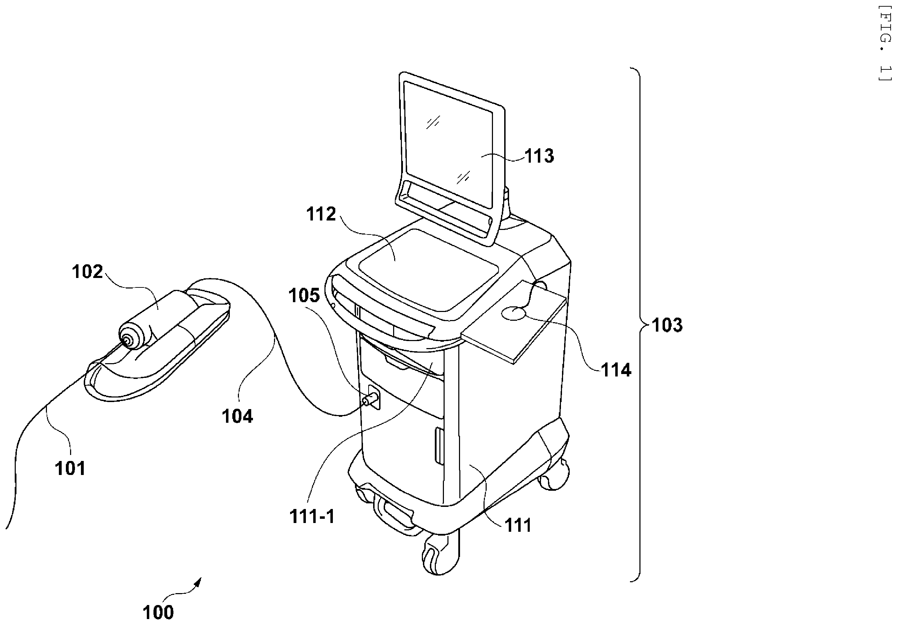

FIG. 1 illustrates an example of an overall configuration of an optical tomography apparatus for diagnosis (hereinafter, simply imaging apparatus for diagnosis) 100 according to an embodiment of the present invention. An apparatus using a wavelength swept light source is taken by way of example in the embodiment; however, since the present invention is applicable to any apparatus using a single wavelength light source, it would be appreciated that the use of the wavelength swept light source is given only for illustrated purposes.

The imaging apparatus for diagnosis 100 is composed by a catheter 101, an MDU part 102, and an operation control apparatus 103, and the MDU part 102 and the operation control apparatus 103 are connected to each other by a cable 104 via a connector 105. This cable 104 accommodates therein an optical fiber and various signal lines.

The catheter 101 protects the optical fiber and accommodates therein a drive shaft for transmitting a rotational force. An imaging core, which has a light transmission-reception unit for transmitting light (measurement light) transmitted from the operation control apparatus 100 via the MDU part 102 in the direction generally orthogonal to a central axis of the optical fiber and receiving reflected light from an outside for the transmitted light, is provided on a tip end of this optical fiber.

The MDU part 102 has the structure of FIG. 2 previously described. Briefly, the MDU part 102 holds the catheter 101 via a connector provided on a rear end of the catheter. The drive shaft within the catheter is rotated by driving a motor included in the MDU part 102, thereby making rotatable the imaging core provided on the tip end thereof. Furthermore, the MDU part 102 performs processing for pulling (retreating) the drive shaft within the catheter at a predetermined speed by driving a motor provided in a linear drive unit included therein.

With the abovementioned configuration, the catheter 101 is guided into a patient's blood vessel, and a radial scan motor included in the MDU part 102 is driven to rotate the drive shaft within the catheter 101, thereby making it possible to scan a vascular luminal surface over 360 degrees. Moreover, the MDU part 102 pulls the drive shaft within the catheter 101 at the predetermined speed, thereby performing a scan along an axis of the blood vessel. It is thereby possible to obtain a plurality of tomographic images viewed from within the blood vessel along the axis of the blood vessel and also possible to reconstruct a three-dimensional blood vessel image by connecting those images.

The operation control apparatus 103 has a function to exert an overall control over operation performed by the imaging apparatus for diagnosis 100. The operation control apparatus 103 has, for example, a function to input various set values based on user's (doctor's) instructions, and a function to process data obtained by measurement and display the processed data as tomographic images of an interior of a body cavity.

The operation control apparatus 103 is provided with a main body control unit 111, a printer/DVD recorder 111-1, an operation panel 112, an LCD monitor 113, etc. The main body control unit 111 generates an optical tomographic image. The optical tomographic image is generated by generating data on interference light by interfering the reflected light which has been obtained by the measurement with reference light which has been obtained by separating light from the light source and by processing line data which has been generated on the basis of the interference light.

The printer/DVD recorder 111-1 prints out a processing result of the main body control unit 111 and/or stores the processing result as data. The operation panel 112 is a user interface to which a user inputs various set values and instructions. The LCD monitor 113, which functions as a display apparatus, displays, for example, the tomographic image generated by the main body control unit 111. 114 denotes a mouse that serves as a pointing device (coordinate input device) .

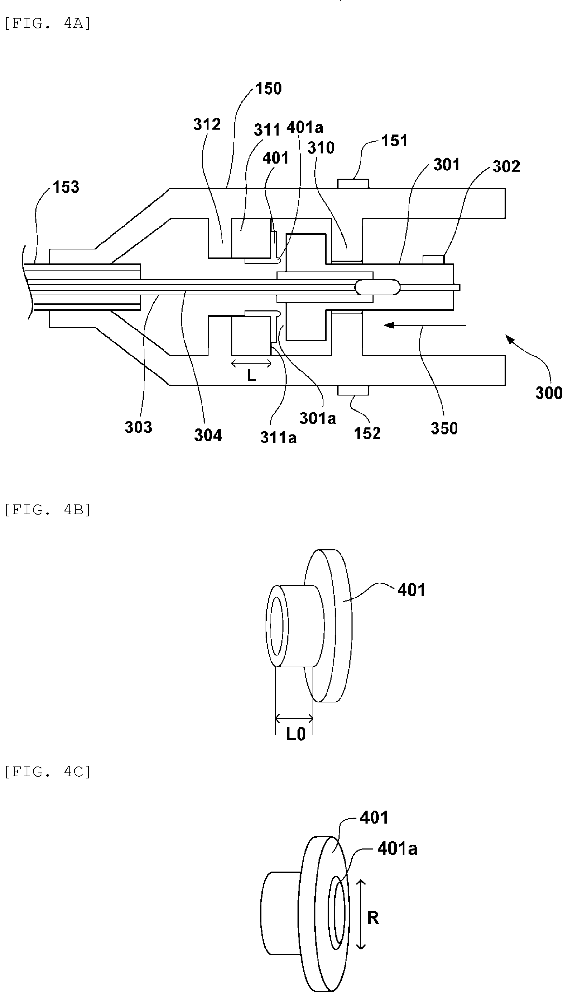

While the general configuration and operation of the imaging apparatus for diagnosis 100 have been described above, the feature of the present embodiment lies in the structure of the connector of the catheter 101 to be connected to the MDU part 102. A structure of the MDU part 102 is assumed to be the same as that shown in FIG. 2, and the structure of the catheter connector 150 according to the embodiment will now be described with reference to FIGS. 4A to 4C. It is noted that similar parts to those of the conventional connector shown in FIG. 3 are denoted by the same reference symbols and the reference symbols are not described herein.

FIG. 4A illustrates a cross-sectional structural view of the catheter connector 150 according to the embodiment. The catheter connector 150 differs from that shown in FIG. 3 in that a hard ring member 401 is interposed between the elastic body 311 (constituted by silicone rubber) and the knob 301 and are the same in the other respects. This ring member 401 has a cylindrical portion fitted into an inside diameter of the elastic body 311, and a flange portion (see FIG. 4B) for dispersing a force of contact when the ring member 401 contacts the rear end surface 301a of the knob 301. Furthermore, a projection portion 401a along a circumference of the flange portion is provided on a knob 301-side thereof, as shown in FIG. 4C. A diameter R of this projection portion 401a is set to be equal to or close to an innermost diameter of the flange portion. It is noted that a depth L0 of the cylindrical portion of the ring member 411 is sufficiently smaller than a thickness L of the elastic body 311 in the depth direction thereof. Structuring the catheter connector 150 as described above makes it possible to maintain buffering properties by the elastic body 311 even when the knob 301 retreats and the rear end surface 301a of the knob is pressure-contacted to the projection portion 401a of the ring member 401 if the catheter is to be connected to the MDU part 102.

Moreover, POM (Polyoxymethylene) is used as a material of this ring member 401 according to the embodiment. As a result, the material of the ring member 401 can reduce per se a frictional force between the knob 301 and the elastic body 311. Furthermore, providing the projection portion 401a along the innermost diameter of the ring member 401 makes it possible to further reduce a frictional torque between the elastic body 311 and the knob 301. As a result, a frictional torque between the ring member 401 and the knob 301 can be made sufficiently lower than a rotation suppression force of the knob engagement portion 206 when the MDU part 102 is not driven. Namely, when the catheter connector 150 is inserted into the MDU connector 204, it is the knob 301-side of the catheter connector 150 that rotates, so that it is possible to greatly alleviate a burden on a contact point between the projection portion 302 of the knob 301 and the guide walls 207 of the knob engagement portion 206 as compared with a conventional technique.

It is noted that the example where the POM is used as the material of the ring member 401 according to the embodiment has been described; in conclusion, however, the material of the ring member is not limited to the POM as long as the material thereof satisfies a condition that the frictional force between the ring member 401 and the knob 301 is lower than the rotation suppression force of the knob engagement portion 206 when the MDU part 102 is not driven. Nevertheless, PBT (Poly Butylene Terephthalate resin) is generally used for the knob 301 and the POM is preferably used for the material of the ring member 401 since the POM is sufficiently lower in frictional resistance than the PBT and is inexpensive.

As described so far, the catheter according to the present embodiment can be configured so that the side thereof which operates to rotate for ensuring accurate optical coupling is the knob side thereof within the catheter which is low in resistance during rotation when the catheter is connected to the MDU part which the imaging apparatus for diagnosis has. It is, therefore, possible to alleviate the burden on the MDU part 102 during the connection as compared with a conventional structure and improve a working ratio thereof.

### [Illustrative example not falling under the scope of the claims]

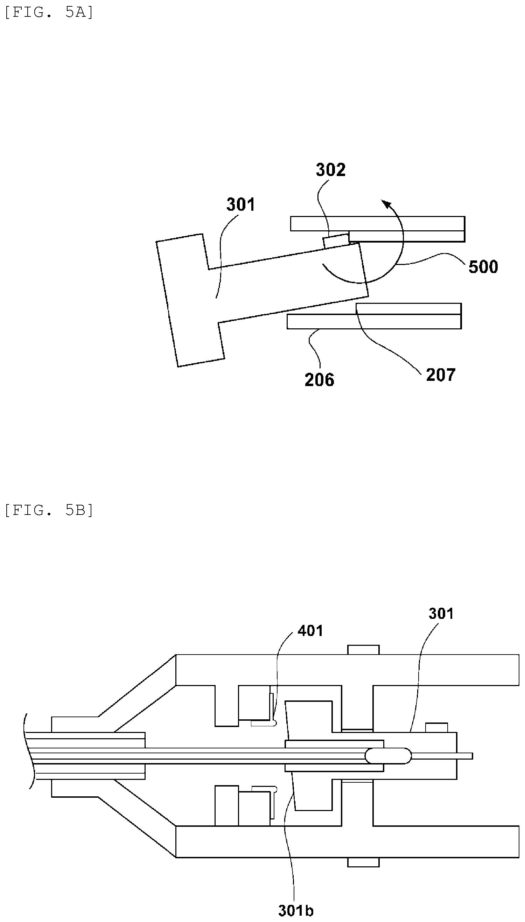

Furthermore, the inventor of the present invention paid attention to the generation of a rotational torque about the contact point as indicated by an arrow 500 of FIG. 5A from a deviation between a line of force generated at the contact point and a line of force of a resultant force received by the knob 301 from the ring member 401 when the projection portion 302 of the knob 301 abuts the guide walls 207 of the knob engagement portion 206 if the catheter is to be connected to the MDU part. While FIG. 5A illustrates as if the knob engagement portion 206 permits the knob 301 to enter the knob engagement portion 206 in a state where the knob 301 is inclined, this is merely an exaggeration for helping understand the disclosure and FIG. 5A does not illustrate that such a clearance is present in the knob engagement portion 206.

The generation of the torque 500 as shown indicates that the knob 301 enters the knob engagement portion 206 in a slightly inclined state. When the catheter 101 is attached to the MDU part 102, an attachment force therefor is received by a contact part where the knob 301 contacts the knob engagement portion 206 and a shear force is generated in the projection portion 302 of the knob 301. When the knob 301 is in the inclined state, a cross-sectional area receiving this shear force is smaller than that when the catheter is attached to the MDUpart 102 in a state where the knob 301 is not inclined. A high shear stress might be thereby generated in the projection portion 302 of the knob 301 and the projection portion 302 of the knob 301 might be broken or deformed.

The inventor of the present invention considered that it would be desirable to provide a structure for eliminating the torque 500 or generating an inverse rotational torque in order to make larger the cross-sectional area receiving the aforementioned shear force and to reduce the shear stress. Fortunately, it is clear from FIG. 5A that a rotation center of the torque 500 is defined by a position on the circumference of the knob 301 where the projection portion 302 is provided. For that reason, a surface, which abuts the ring member 401, of the knob 301 is formed into a surface such that a flange portion of the knob 301 becomes the thickest in the same direction as the projection portion 302 and becomes the thinnest in the direction opposite to the projection portion 302. Structuring the knob 301 as described above enables an upper end of a surface 301b to contact first the ring member 401 or the projection portion 401a in FIG. 5B when the catheter is pressed into the MDU part 102 to retreat the knob 301, so that a state where the torque 500 shown previously is not present at all can be created or the torque in the opposite direction can be applied to the knob 301. As a result, it is possible to secure a large cross-sectional area which receives the abovementioned shear force in the contact part between the projection portion 302 of the knob 301 and the guide walls 207 of the knob engagement portion 206; therefore, it is eventually possible to reduce the shear stress and further alleviate the burden on the projection portion 302 of the knob 301.

The embodiments according to the present invention have been described so far. In the present embodiments, the example where the present invention is applied to the imaging apparatus for diagnosis using optical coherence using the OCT or OFDI has been described. An apparatus that has not only a light transmission/reception unit but also an ultrasound transmission/reception unit provided in an imaging core and generating a tomographic image using optical coherence and a tomographic image using an ultrasound wave at one scan has recently been developed; therefore, the present invention may be applied to such an apparatus.

The present invention is not limited to the abovementioned embodiments but can be variously changed and modified to include everything within the scope of the appended claims Hence, the following claims are attached in order to make the scope of the present invention public.

## Claims

1. An optical imaging apparatus (100) for diagnosis comprising
a drive unit (102) which functions as a power source for the rotation and axial movement of a drive shaft (303), and
a catheter (101) comprising said drive shaft (303) and a connector (150) for connecting the catheter (101) to the drive unit (102), wherein
the connector (150) comprises
a fiber end holding part exposing and holding an end surface of an optical fiber (304) in order to optically connect the catheter (101) to the drive unit (102);
an elastic body (311) buffering a load when the catheter (101) is connected to the drive unit (102); and
a member (401), provided between the elastic body (311) and the fiber end holding part, abuts the fiber end holding part when the fiber end holding part retreats due to pressing of the connector (150) into the drive unit (102) and is fixed to the elastic body (311),
**characterized in that**
a frictional force between the member (401) and the fiber end holding part is lower than a rotation suppression force of an engagement portion (206) of the drive unit (102) when the drive unit (102) is not driven and the connector (150) is pressed into the drive unit (102), the engagement portion (206) being configured to engage the fiber end holding part.

2. The optical imaging apparatus (100) for diagnosis according to claim 1, wherein
the fiber end holding part is provided with a projection portion (302) guided into a guide groove (208) provided in the drive unit (102), and
the optical fiber (304) has an end surface inclined with respect to a surface orthogonal to the axial direction of the optical fiber (304).

3. The optical imaging apparatus (100) for diagnosis according to claim 1 or 2, wherein the member (401) comprises a cylindrical portion to be fitted into the elastic body (311); a flange portion for receiving a force generated when the member (401) contacts the fiber end holding part on a surface thereof; and a projection portion (401a) provided on a surface, toward the fiber end holding part, of the flange portion, which has an innermost cylindrical shape, and which contacts the fiber end holding part.

4. The optical imaging apparatus (100) for diagnosis according to any one of claims 1 to 2, wherein the member (401) comprises polyoxymethylene.

## Patentansprüche

1. Optische Bildgebungsvorrichtung (100) für die Diagnose, umfassend
eine Antriebseinheit (102), die als Energiequelle für die Drehung und axiale Bewegung einer Antriebswelle (303) fungiert, und
einen Katheter (101), der die Antriebswelle (303) und ein Verbindungsstück (150) zum Verbinden des Katheters (101) mit der Antriebseinheit (102) umfasst, wobei
das Verbindungsstück (150) umfasst
ein Faserende-Halteteil, das eine Endfläche einer optischen Faser (304) freilegt und hält, um den Katheter (101) optisch mit der Antriebseinheit (102) zu verbinden;
einen elastischen Körper (311), der eine Belastung puffert, wenn der Katheter (101) mit der Antriebseinheit (102) verbunden ist; und
ein Element (401), das zwischen dem elastischen Körper (311) und dem Faserende-Halteteil vorgesehen ist, welches an dem Faserende-Halteteil anliegt, wenn sich das Faserende-Halteteil aufgrund des Eindrückens des Verbindungsstücks (150) in die Antriebseinheit (102) zurückzieht, und an dem elastischen Körper (311) befestigt ist,
**dadurch gekennzeichnet, dass**
eine Reibungskraft zwischen dem Element (401) und dem Faserende-Halteteil geringer ist als eine Drehungsunterdrückungskraft eines Eingriffsabschnitts (206) der Antriebseinheit (102), wenn die Antriebseinheit (102) nicht angetrieben wird und das Verbindungsstück (150) in die Antriebseinheit (102) eingedrückt wird, wobei der Eingriffsabschnitt (206) so konfiguriert ist, dass er mit dem Faserende-Halteteil in Eingriff kommt.

2. Optische Bildgebungsvorrichtung (100) zur Diagnose nach Anspruch 1, wobei
das Faserende-Halteteil mit einem Vorsprungsabschnitt (302) versehen ist, der in einer Führungsnut (208) geführt wird, die in der Antriebseinheit (102) vorgesehen ist, und
die optische Faser (304) eine Endfläche aufweist, die in Bezug auf eine Fläche senkrecht zur axialen Richtung der optischen Faser (304) geneigt ist.

3. Optische Bildgebungsvorrichtung (100) zur Diagnose nach Anspruch 1 oder 2, wobei das Element (401) einen zylindrischen Abschnitt umfasst, um in den elastischen Körper (311) eingepasst zu werden; einen Flanschabschnitt zum Aufnehmen einer Kraft, die erzeugt wird, wenn das Element (401) mit dem Faserende-Halteteil auf einer Oberfläche davon in Kontakt kommt; und einen Vorsprungsabschnitt (401a), der in Richtung des Faserende-Halteteils, auf einer Oberfläche des Flanschabschnitts vorgesehen ist, der eine innerste zylindrische Form aufweist und mit dem Faserende-Halteteil in Kontakt kommt.

4. Optische Bildgebungsvorrichtung (100) zur Diagnose nach einem der Ansprüche 1 bis 2, wobei das Element (401) Polyoxymethylen umfasst.

## Revendications

1. Appareil d'imagerie optique (100) de diagnostic comprenant
une unité de commande (102) qui sert de source d'alimentation pour la rotation et le mouvement axial d'une tige de commande (303), et
un cathéter (101) comprenant ladite tige de commande (303) et un connecteur (150) pour connecter le cathéter (101) à l'unité de commande (102), dans lequel le connecteur (150) comprend
une partie de maintien d'extrémité de fibre exposant et maintenant une surface d'extrémité d'une fibre optique (304) afin de connecter optiquement le cathéter (101) à l'unité de commande (102) ;
un corps élastique (311) amortissant une charge lorsque le cathéter (101) est connecté à l'unité de commande (102) ; et
un organe (401), disposé entre le corps élastique (311) et la partie de maintien d'extrémité de fibre, vient en butée contre la partie de maintien d'extrémité de fibre lorsque la partie de maintien d'extrémité de fibre se rétracte du fait de l'enfoncement du connecteur (150) dans l'unité de commande (102) et est fixé au corps élastique (311),
**caractérisé en ce que**
une force de friction entre l'organe (401) et la partie de maintien d'extrémité de fibre est inférieure à une force de suppression de rotation d'une partie de mise en prise (206) de l'unité de commande (102) lorsque l'unité de commande (102) n'est pas entraînée et le connecteur (150) est enfoncé dans l'unité de commande (102), la partie de mise en prise (206) étant configurée pour venir en prise avec la partie de maintien d'extrémité de fibre.

2. Appareil d'imagerie optique (100) de diagnostic selon la revendication 1, dans lequel
la partie de maintien d'extrémité de fibre est dotée d'une partie saillante (302) guidée dans une rainure de guidage (208) prévue dans l'unité de commande (102), et la fibre optique (304) présente une surface d'extrémité inclinée par rapport à une surface perpendiculaire à la direction axiale de la fibre optique (304).

3. Appareil d'imagerie optique (100) de diagnostic selon la revendication 1 ou 2, dans lequel l'organe (401) comprend une partie cylindrique à insérer dans le corps élastique (311) ; une partie de bride pour recevoir une force générée lorsque l'organe (401) entre en contact avec la partie de maintien d'extrémité de fibre sur une surface de celui-ci ; et une partie saillante (401a) disposée sur une surface, vers la partie de maintien d'extrémité de fibre, de la partie de bride, qui présente une forme cylindrique la plus intérieure, et qui entre en contact avec la partie de maintien d'extrémité de fibre.

4. Appareil d'imagerie optique (100) de diagnostic selon l'une quelconque des revendications 1 à 2, dans lequel l'organe (401) comprend du polyoxyméthylène.
